# EUROPÄISCHE PATENTSCHRIFT

(11) **EP 1 120 404 B1**
(45) Veröffentlichungstag und Bekanntmachung des Hinweises auf die Patenterteilung: **06.04.2005**
(21) Anmeldenummer: 01101050.1
(22) Anmeldetag: 18.01.2001
(51) Int. Cl.: C07C 253/30, C07C 255/16

(54) **Herstellung von Hydroxypropionitril**
Preparation of hydroxypropionitrile
Préparation d'hydroxypropionitrile

(30) Priorität: 25.01.2000 CH 1442000
(43) Veröffentlichungstag der Anmeldung: 01.08.2001
(73) Patentinhaber: DSM IP Assets B.V., 6411 TE Heerlen (NL)
(72) Erfinder: Burdet, Bruno, 68390 Baldershiem (FR); Riegl, Johann, Verstorben (DE); Ruettimann, August, 4144 Arlesheim (CH)
(74) Vertreter: Schwander, Kuno Josef, Dr.

(56) Entgegenhaltungen:
- DE-B- 1 189 975
- DATABASE CA [Online] CHEMICAL ABSTRACTS SERVICE, COLUMBUS, OHIO, US; INOMATA, MASASANE ET AL: "Preparation of ethylene cyanohydrin by hydration and pyrolysis of acrylonitrile" retrieved from STN Database accession no. 112:55014 XP002233682 -& JP 01 160949 A (MITSUI TOATSU CHEMICALS, INC., JAPAN) 23. Juni 1989 (1989-06-23)
- DATABASE CA [Online] CHEMICAL ABSTRACTS SERVICE, COLUMBUS, OHIO, US; INOMATA, MASASANE ET AL: "Preparation of ethylene cyanohydrin by thermal decomposition of bis(cyanoethyl) ether" retrieved from STN Database accession no. 111:133654 XP002233683 -& JP 01 090160 A (MITSUI TOATSU CHEMICALS, INC., JAPAN) 6. April 1989 (1989-04-06)
- DATABASE CA [Online] CHEMICAL ABSTRACTS SERVICE, COLUMBUS, OHIO, US; DAICEL CHEMICAL INDUSTRIES, LTD., JAPAN: "Ethylene cyanohydrin" retrieved from STN Database accession no. 100:120539 XP002233684 -& JP 58 185550 A (DAICEL CHEMICAL INDUSTRIES, LTD., JAPAN) 29. Oktober 1983 (1983-10-29)

## Beschreibung

Die vorliegende Erfindung betrifft ein Verfahren zur Herstellung von 3-Hydroxypropionitril, welches ein wichtiges Zwischenprodukt im Verfahren zur Herstellung von Panthenol darstellt. Das Endprodukt, insbesondere dessen d(+)- Isomer, ist ein wertvolles Mittel zur Behandlung von Dermatosen, Verbrennungen und infektiösen Geschwüren sowie ein wertvoller Zusatzstoff in Haarwässern und anderen Kosmetika.

Verschiedene Methoden zur Herstellung von 3-Hydroxypropionitril sind aus der Literatur bekannt. Beispielsweise wurde bereits 1863 die Umsetzung von 2-Chlorethanol mit einem Alkalicyanid zu 3-Hydroxypropionitril (auch als "Ethylencyanhydrin" bekannt) beschrieben [Annalen der Chemie und Pharmacie 128, 1 (1863)]. Spätere Publikationen, wie beispielsweise Org. Synth. 3, 57 (1923), J.Soc. Chem. Ind. 67, 458 (1948) sowie die US-Patentschrift 2.311.636 betreffen die Optimierung dieser grundlegenden Synthese. Trotz der angeblich erreichten hohen Ausbeute ist das Verfahren wegen des relativ hohen Anschaffungspreises des Ausgangsmaterials 2-Chlorethanol sowie der schwierig zu kontrollierenden Reaktionsexothermie kommerziell uninteressant. Zudem ist sowohl die Salzbelastung wie auch der Aufarbeitungsaufwand gross. Ueber eine erfolgreiche Herstellung von 3-Hydroxypropionitril durch Anlagerung von Blausäure an Ethylenoxid wurde bereits um 1930 in verschiedenen Deutschen Patentschriften berichtet, und zwar in den Deutschen Patentschriften 561.397, 570.031 sowie 577.686. Später wurde in der US-Patentschrift 2.653.162 eine Optimierung dieses Anlagerungsverfahrens beschrieben, bei der ein Carbonsäure-Natriumsalz-Harz als schwache Base und Wasser als Lösungsmittel verwendet werden und die Reaktion bei 45 - 50°C erfolgt. Noch spätere Offenbarungen der Herstellung von 3-Hydroxypropionitril durch Anlagerung von Blausäure an Ethylenoxid erscheinen in der Deutschen Offenlegungsschrift 4.304.002 sowie in der US-Patentschrift 5.268.499. Die beiden Ausgangsmaterialien sind bezüglich Toxizität und Handhabung sehr problematisch und kommen deswegen für eine zeitgemässe Herstellung von 3-Hydroxypropionitril nicht mehr in Frage.

Die Herstellung des bekannten 1 : 1 - Additionsproduktes von Wasser und Acrylnitril, d.h. von 3-Hydroxypropionitril, in Gegenwart eines basischen Katalysators bereitet gewisse Schwierigkeiten, da das Produkt mit weiterem Acrylnitril zum Kondensationsprodukt Bis(cyanoethyl)ether, NC(CH₂)₂O(CH₂)₂CN, reagiert: siehe die US-Patentschrift 2.579.580. Gemäss der US-Patentschrift 3.024.267 und der japanischen Patentpublikation (Kokai) 196.850/1984 werden etwas bessere Ausbeuten am gewünschten 3-Hydroxypropionitril nur erreicht, wenn die Umsetzung in einem grossen Ueberschuss von Wasser, d.h. in grosser Verdünnung, bzw. gemäss J.Org. Chem. USSR 1987, 1087 in Gegenwart einer grössen Menge an Base, durchgeführt wird. Diese Massnahmen sind jedoch unwirtschaftlich, und zwar insbesondere wegen der unvermeidlichen aufwendigen Aufarbeitung, Verdampfung von Wasser und Salzbelastung. Zudem werden auch bei der grossen Verdünnung stets kleinere bis mittlere Mengen an Bis(cyanoethyl)ether gebildet.

Als mögliche Lösung obiger Probleme ist in der Deutschen Patentschrift 2.655.794 die Umsetzung wässrigen Formaldehyds mit Acrylnitril in Gegenwart von stark basischem Amberlyst® IRA-400, OH⁻-Form, bei etwa 40°C vorgeschlagen; die angegebene 75%ige Ausbeute von 3-Hydroxypropionitril ist jedoch auch in diesem Fall mässig.

Die oben geschilderten Verfahren zur unmittelbaren Herstellung von 3-Hydroxypropionitril weisen offensichtliche Nachteile auf.

Aus den Japanischen Patentpublikationen (Kokai; JP) 160.949/1989, 90.160/1989 und 185550/1983 ist bekannt, dass sich Bis(cyanoethyl)ether aus Acrylnitril und Wasser in Gegenwart eines stark basischen Ionenaustauschers herstellen lässt, und dass das Produkt anschliessend mittels eines basischen Katalysators zum gewünschten 3-Hydroxypropionitril und zu Acrylnitril pyrolysiert werden kann. Auch aus früherem Stand der Technik ist die basenkatalysierte Herstellung von Bis(cyanoethyl)ether aus Acrylnitril und Wasser bekannt: siehe Org. Reactions 5, 79 (1945; Uebersichtsartikel), J.A.C.S. 65, 23 (1943), ibid. 67, 1996 (1945), Ind. Eng. Chem. 44, 1388 (1952), Deutsche Patentschriften 731.708 und 1.189.975 sowie US-Patentschriften 2.382.036, 2.448.979 und 2.816.130. Als Katalysator für diese 2 : 1 - Additionsreaktion wird im allgemeinen eine starke Base, z.B. Natriumhydroxid, aber auch ein stark basischer Amberlyst®-Katalysator, wie Amberlyst® IRA-400, OH⁻-Form, verwendet. Unabhängig von dem Verhältnis Acrylnitril: Wasser, der Reaktionstemperatur und der jeweils verwendeten starken Base werden zumindest kleinere Mengen 3-Hydroxypropionitril gebildet. Mittels stark basischer Katalysatoren, z.B. Amberlyst® IRA-400, HO⁻-Form konnten nach diesem Stand der Technik Ausbeuten von bis zu etwa 77% erreicht werden. Die in den oben erwähnten Japanischen Patentpublikationen beschriebene Spaltung des Bis(cyanoethyl)ethers erfolgt unter Verwendung eines basischen Katalysators, und zwar insbesondere von wässrigem oder wässrigmethanolischem Kaliumhydroxid/Dikaliumphosphat bei 72 - 100°C / 5 mm Hg mit etwa 85%iger Ausbeute (JP 160.949/1989, JP 90.160/1989) bzw. von Tetraethylammoniumacetat bei 100 - 105°C / 10 mm Hg mit etwa 86%iger Ausbeute (JP 185.550/1983).

Es ist nun ein neues Verfahren zur Herstellung von 3-Hydroxypropionitril gefunden worden, welches die Umsetzung von Wasser mit Acrylnitril zu einem Gemisch des erwünschten Produktes und des Kondensationsproduktes Bis(cyanoethyl)ether und die Pyrolyse dieses Mischproduktes zu weiterem 3-Hydroxypropionitril beinhaltet, wobei sowohl die erwähnte Umsetzung wie auch die Pyrolyse unter ganz besonderen basenkatalytischen und weiteren Bedingungen durchgeführt werden und unreagierte Reaktionsteilnehmer, insbesondere Acrylnitril und das den Katalysator enthaltende Wasser, gewünschtenfalls in das Reaktionssystem zwecks Wiederverwendung zurückgeführt werden können. Das ganze mehrstufige Verfahren ermöglicht eine insgesamt überraschend ökonomische Herstellung von 3-Hydroxypropionitril, zumal dieses Produkt in der ersten Verfahrensstufe nur teilweise gebildet, allerdings in der vorletzten (Pyrolyse-) Stufe in hoher Ausbeute erhalten wird.

Bei dem erfindungsgemässen Verfahren zur Herstellung von 3-Hydroxypropionitril handelt es sich um ein Verfahren, welches von Acrylnitril und Wasser ausgeht und dadurch gekennzeichnet ist, dass man
(a) in einem Temperaturbereich von 80°C bis 150°C und bei einem Druck von 1 bar (0,1 MPa) bis 5 bar (0,5 MPa) Acrylnitril mit Wasser in einem Molverhältnis von 1 : 0,5 bis 1 : 20 in Gegenwart einer schwachen Base, welche eine anorganische oder organische Base mit einem pKₐ-Wert von 8 bis 12 ist, solange umsetzt, bis ein Umsatz im Bereich von 40% bis 80% erreicht worden ist, um ein zweiphasiges organisch-wässriges Gemisch zu erhalten, dessen organische Phase hauptsächlich aus Bis(cyanoethyl)ether, 3-Hydroxypropionitril sowie unreagiertem Acrylnitril besteht, und dessen wässrige Phase hauptsächlich aus einer wässrigen Lösung der schwachen Base besteht;
(b) nach Abkühlung des in (a) erhaltenen Gemisches dessen wässrige Phase abtrennt;
(c) von der nach (b) verbleibenden organischen Phase das Acrylnitril abdestilliert, um ein hauptsächlich aus Bis(cyanoethyl)ether und 3-Hydroxypropionitril bestehendes Gemisch zu erhalten;
(d) das in (c) erhaltene Gemisch einer Pyrolyse in einem Temperaturbereich von 120°C bis 160°C und bei einem verminderten Druck von 5 mbar (0,5 kPa) bis 500 mbar (50 kPa) in Gegenwart eines basichen Katalysators unterwirft, welcher von der Gruppe Calcium-, Magnesium-, Strontium-, Titan-, Eisen- und Zinkoxide; Alkalimetallacetate; Alkalimetallformiate; Alkalimetall- und Bariumcarbonate; Alkalimetallbicarbonate; Calcium- und Kupferhydroxide; Di- und Trinatriumphosphate; Natriumfluorid; Natriumsilikat; und hochsiedende Trialkylamine ausgewählt ist, um ein hauptsächlich aus 3-Hydroxypropionitril und Acrylnitril bestehendes Gemisch zu erhalten; und
(e) von dem in (d) erhaltenen Gemisch das gewünschte 3-Hydroxypropionitril durch fraktionierte Destillation isoliert.

Gewünschtenfalls, und sogar vorzugsweise, wird die in der Verfahrensstufe (b) abgetrennte wässrige Phase, welche den Hauptteil der in der Stufe (a) verwendeten Base enthält, in die Stufe (a) eines kontinuierlich durchgeführten Gesamtverfahrens zurückgeführt, um dort mit weiterem Acrylnitril zu reagieren. Ebenfalls wird gewünschtenfalls und vorzugsweise das in der Verfahrensstufe (c) und/oder in der Verfahrensstufe (e) abdestillierte Acrylnitril in die Stufe (a) eines solchen Verfahrens zurückgeführt. Ein derartiges Verfahren, welches solche Rezyklisierung der basischen wässrigen Phase sowie des abdestillierten Acrylnitrils aufweist, kann wie folgt schematisch dargestellt werden:

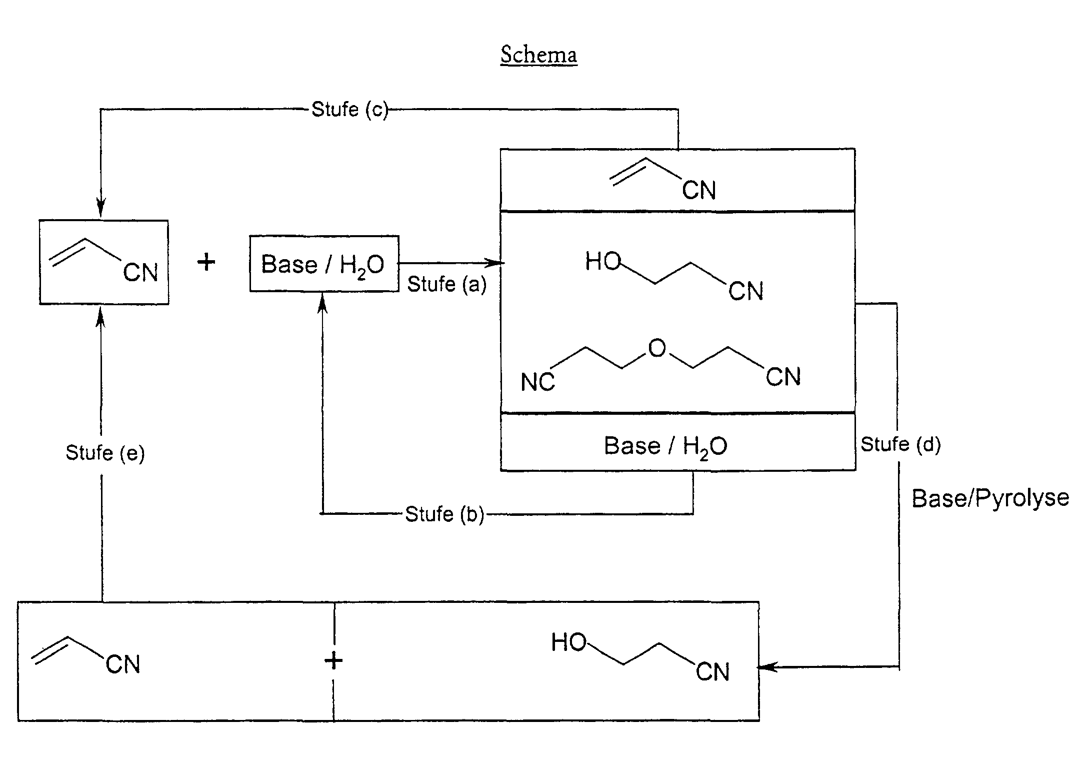

Die in der Verfahrensstufe (a) verwendete "schwache Base", d.h. die anorganische oder organische Base deren pKₐ-Wert etwa 8 bis etwa 12 beträgt, ist als anorganische Base zweckmässigerweise ein Alkalimetallcarbonat, z.B. Natrium- oder Kaliumcarbonat, ein Alkalimetallbicarbonat, z.B. Natrium- oder Kaliumbicarbonat, oder ein Gemisch von zwei oder mehreren dieser anorganischen Basen, z.B. ein Gemisch von Natriumcarbonat und -bicarbonat, bzw. als organische Base zweckmässigerweise ein niederes Trialkylamin oder ein 4-Dialkylaminopyridin. Sowohl bei "niederes Trialkylamin" wie auch bei "4-Dialkylamino-pyridin" ist unter dem Ausdruck "alkyl" insbesondere eine C₁₋₆-Alkyl-gruppe" zu verstehen. Beispiele von niederen Trialkylaminen und 4-Dialkylamino-pyridinen sind Triethylamin und Ethyldiisopropylamin bzw. 4-dimethylamino-pyridin. Vorzugsweise wird als schwache Base Natriumcarbonat, Kaliumcarbonat, ein Gemisch von Natriumcarbonat und -bicarbonat, oder ein Gemisch von Kaliumcarbonat und -bicarbonat verwendet.

Bezogen auf die Menge eingesetzten Acrylnitrils beträgt die Menge der eingesetzten schwachen Base zweckmässigerweise 0,1 bis 5 Mol%, vorzugsweise 0,5 bis 2 Mol%.

Die Umsetzung des Acrylnitrils mit dem Wasser erfolgt in der Verfahrensstufe (a) im allgemeinen im Temperaturbereich von etwa 80°C bis etwa 150°C, vorzugsweise bei Temperaturen im Bereich von 100°C bis 130°C, und im allgemeinen bei einem Druck von etwa 1 bar (0,1 MPa) bis etwa 5 bar (0,5 MPa), vorzugsweise von 1 bar (0,1 MPa) bis 3 bar (0,3 MPa).

Das Molverhältnis Acrylnitril: Wasser beträgt im allgemeinen etwa 1 : 0,5 bis etwa 1 : 20, vorzugsweise 1 : 3 bis 1 : 8, ganz bevorzugt 1 : 2 bis 1 : 4.

Unter dem in der Definition der Verfahrensstufe (a) vorkommenden Ausdruck "hauptsächlich aus Bis(cyanoethyl)ether, 3-Hydroxypropionitril sowie unreagiertem Acrylnitril" ist zu verstehen, dass diese drei Bestandteile der diesbezüglichen organischen Phase gewichtsmässig mindestens 90% ausmachen. In derselben Definition bedeutet der Ausdruck "hauptsächlich aus einer wässrigen Lösung der schwachen Base", dass die Summe der Gewichte der schwachen Base und des Wassers, in dem sie gelöst ist, mindestens 70% des Gewichtes der wässrigen Phase beträgt. In Bezug auf die Verfahrensstufe (c) ist der darin vorkommenden Ausdruck "hauptsächlich aus Bis(cyanoethyl)ether und 3-Hydroxypropionitril" so zu verstehen, dass diese zwei Bestandteile des diesbezüglichen Gemisches gewichtsmässig mindestens 80% ausmachen. Schliesslich ist unter dem in der Definition der Verfahrensstufe (d) vorkommenden Ausdruck "hauptsächlich aus 3-Hydroxypropionitril und Acrylnitril" zu verstehen, dass diese zwei Bestandteile des diesbezüglichen Gemisches gewichtsmässig mindestens 90% ausmachen.

Die Umsetzung (Kondensation) von Acrylnitril mit Wasser in Gegenwart der schwachen Base erfolgt arbeitstechnisch zweckmässig, indem man das Acrylnitril mit einer Lösung der Base in Wasser bei Raumtemperatur in einem Autoklaven zusammenbringt und das so erhaltene (zweiphasige) Gemisch unter Rühren aufheizt. In dem verschlossenen Autoklaven baut sich dabei ein Druck auf, der je nach Temperatur (80°C bis 150°C) etwa 1 bis 5 bar (0,1 bis 0,5 MPa) beträgt.

Der Umsatz wird bei der Verfahrensstufe (a) absichtlich auf den Bereich von 40% bis 80% begrenzt, da ein zu hoher Umsatz zu übermässiger Erzeugung von unerwünschten polymeren Nebenprodukten sowie zunehmend vom ebenfalls unerwünschten Nebenprodukt Acrylamid führt. Dass dabei sich unreagiertes Acrylnitril im Produkt befindet, ist nicht problematisch, da dieses Ausgangsmaterial sehr leicht [in der Verfahrensstufe (c)] wiedergewonnen und rezyklisiert werden kann, und zwar insbesondere in die Stufe (a). Zudem ist es nicht relevant, ob bei der Kondensation das Verhältnis der Produkte Bis(cyanoethyl)ether : 3-Hydroxypropionitril gross oder klein ist, denn das erstere Produkt lässt sich leicht [in der Verfahrensstufe (d)] zum erwünschten 3-Hydroxypropionitril überführen und das letztere Produkt ist unter den erfindungsgemässen Verfahrensbedingungen, insbesondere den Pyrolysebedingungen der Stufe (d), stabil, so dass das hauptsächlich aus den beiden genannten Produkten bestehende Gemisch unmittelbar der Pyrolyse unterworfen werden kann.

Die Begrenzung des Umsatzes bedingt dessen Kontrolle und Bemessung, was zweckmässigerweise durch Gaschromatographie erfolgen kann.

Die Verfahrensstufe (a) wird in der Regel bereits nach etwa 1 bis 2 Stunden beendet.

Vorteilhaft bei der Stufe (a) des gesamten erfindungsgemässen Verfahrens ist unter anderem die weitgehende Unterdrückung der Bildung von unerwünschten polymeren Nebenprodukten sowie vom ebenfalls unerwünschten Nebenprodukt Acrylamid.

Bei der Verfahrensstufe (b) handelt es sich um die Abtrennung der wässrigen Phase von dem in der Verfahrensstufe (a) erhaltenen zweiphasigen organisch-wässrigen Gemisch. Dieses Gemisch wird vor der Abtrennung zweckmässigerweise auf eine Temperatur im Bereich von Raumtemperatur bis etwa +10°C abgekühlt, was entweder ohne aktive Kühlung oder durch Kühlung mit kaltem Wasser, beispielsweise Wasser bei etwa + 5°C, erfolgt. In diesem Temperaturbereich trennen sich die beiden Phasen sehr leicht.

Die wässrige Phase, welche den grössten Teil der als Katalysator verwendeten schwachen Base enthält, kann anschliessend abgetrennt werden, was zweckmässiger-weise nach an sich bekannten Methoden erfolgt.

Vorzugsweise wird die so abgetrennte wässrige Phase anschliessend in die Stufe (a) zuruckgeführt, um nach allfälliger Ergänzung mit schwacher Base mit weiterem, gegebenenfalls auch zum Teil zurückgeführtem Acrylnitril in einem zweiten oder weiteren Zyklus der Verfahrensstufe (a) umgesetzt zu werden.

Die nach erfolgter Durchführung der Verfahrensstufe (b) verbleibende organische Phase, welche hauptsächlich aus Bis(cyanoethyl)ether, 3-Hydroxypropionitril und nicht umgesetztem Acrylnitril besteht, wird dann in der Verfahrensstufe (c) destilliert, um das Acrylnitril von dem Bis(cyanoethyl)ether und 3-Hydroxypropionitril abzutrennen. Dies geschieht zweckmässigerweise, indem man das Acrylnitril mit zum Teil noch gelöstem Wasser über eine Destillationskolonne, z.B. eine verspiegelte Füllkörper-Kolonne, abdestilliert, und zwar bei Temperaturen im Bereich von etwa 60°C bis etwa 100°C und bei einem Druck von etwa 200 mbar (20 kPa) bis etwa 10 mbar (1 kPa). Dabei wird das abdestillierte Acrylnitril laufend in einer geeignet gekühlten Vorlage aufgefangen.

Das so abgetrennte Acrylnitril wird vorzugsweise in die Stufe (a) zurückgeführt, um nach allfälliger Ergänzung mit weiterem Acrylnitril mit Wasser in Gegenwart einer schwachen Base in einem zweiten oder weiterem Zyklus der Verfahrensstufe (a) umgesetzt zu werden.

Das nach erfolgter Durchführung der Verfahrensstufe (c) verbleibende Gemisch von (hauptsächlich) Bis(cyanoethyl)ether und 3-Hydroxypropionitril wird anschliessend in der Verfahrensstufe (d) pyrolysiert, wobei der Bis(cyanoethyl)ether unter der basischen Katalyse in 3-Hydroxypropionitril und Acrylnitril gespalten wird, ohne dass das sich ebenfalls im Gemisch befindende 3-Hydroxypropionitril selber reagiert; dieses gewünschte Produkt bleibt unter den Bedingungen der Pyrolyse unverändert, was einen überraschenden Vorteil des erfindungsgemässen Verfahrens darstellt.

Der in der Definition der Verfahrensstufe (d) vorkommende Ausdruck "Alkalimetall" bedeutet jeweils Lithium, Natrium oder Kalium. Falls als basischer Katalysator ein hochsiedendes Trialkylamin verwendet wird, ist dies ein Trialkylamin, dessen Siedepunkt bei Normaldruck höher ist als etwa 150°C; Beispiele sind Trioctylamin und Tridodecylamin.

Unter den verwendbaren basischen Katalysatoren sind Calciumoxid, Magnesiumoxid, die Alkalimetallacetate, die Alkalimetallformiate, die Alkalimetallcarbonate, Bariumcarbonat, die Alkalimetallbicarbonate, Calciumhydroxid, Trinatriumphosphat sowie Natriumfluorid bevorzugt. Besonders bevorzugte basische Katalysatoren sind Calciumoxid, Natriumacetat und Kaliumacetat.

Bezogen auf die Gesamtmenge des in der Verfahrensstufe (c) erhaltenen und in der Verfahrensstufe (d) eingesetzen Gemisches von Bis(cyanoethyl)ether und 3-Hydroxypropionitril beträgt die Menge des eingesetzten basischen Katalysators zweckmässigerweise 0,05 bis 10 Gewichtsprozent, vorzugsweise 0,1 bis 3 Gewichtsprozent.

Die Pyrolyse der Verfahrensstufe (d) erfolgt im allgemeinen im Temperaturbereich von etwa 120°C bis etwa 160°C, vorzugsweise bei Temperaturen im Bereich von 130°C bis 150°C, und im allgemeinen bei einem verminderten Druck von etwa 5 mbar (0,5 kPa) bis etwa 500 mbar (50 kPa), vorzugsweise von 10 mbar (1 kPa) bis 400 mbar (40 kPa).

Es entsteht durch die Pyrolyse ein Gemisch von hauptsächlich 3-Hydroxypropionitril und Acrylnitril im Molverhältnis von etwa 1 : 1. Die Dauer der Pyrolyse, durch welche ein solches Gemisch erhalten wird, hängt von der Ansatzgrösse ab und beträgt in der Regel mindestens eine Stunde. Dabei werden nur sehr wenig Acrylamid und polymere Produkte als Nebenprodukte erzeugt, was einen weiteren Vorteil des erfindungsgemässen Verfahrens darstellt.

Die Pyrolyse kann arbeitstechnisch derart erfolgen, indem man gleichzeitig mit der Pyrolyse die sich bildenden Produkte 3-Hydroxypropionitril und Acrylnitril unter Verwendung einer Destillationskolonne kontinuierlich abdestilliert und in einer gekühlten Vorlage auffängt. Als Alternative kann man die gleichzeitig mit der Pyrolyse erfolgende Destillation selektiv durchführen, indem man die beiden Produkte je einzeln durch geeignete Kühlung selektiv kondensiert, und zwar erst das 3-Hydroxypropionitril und dann das Acrylnitril. Auf diese Weise wird die fraktionierte Destillation der Verfahrensstufe (e) effektiv vorverlegt, wobei es sich um eine Kombination der Verfahrensstufen (d) und (e) handelt. Dies stellt eine Modifikation des erfindungsgemässen Verfahrens dar.

Falls man in der Verfahrensstufe (d) das Gemisch von 3-Hydroxypropionitril und Acrylnitril erhalten hat, wird in der letzten Verfahrensstufe, (e), das gewünschte 3-Hydroxypropionitril durch fraktionierte Destillation isoliert. Gewünschtenfalls - und vorzugsweise - wird das dabei erhaltene Acrylnitril in die Stufe (a) zurückgeführt und wiederverwendet, und zwar analog der Situation, welche oben im Zusammenhang mit der Durchführung der früheren Stufe (c) beschrieben ist.

Die fraktionierte Destillation gemäss der Verfahrensstufe (e) erfolgt zweckmässigerweise zunächst einmal bei einer Badtemperatur von etwa 50°C bis etwa 120°C und einem Druck von etwa 180 mbar (18 kPa) bis etwa 10 mbar (1 kPa), wobei der Hauptteil des Acrylnitrils abdestilliert wird, und danach bei einer Badtemperatur von etwa 120°C bis etwa 150°C und einem Druck von etwa 10 mbar (1 kPa) und unterhalb, wobei der Hauptteil des gewünschten 3-Hydroxypropionitrils [Siedepunkt etwa 99-102°C/10 mbar (1 kPa) ] erhalten wird. Als Destillationskolonne kann beispielsweise eine verspiegelte Füllkörper-Kolonne verwendet werden.

Auf diese Weise erhält man 3-Hydroxypropionitril in sehr guter Reinheit und in einer Ausbeute, die bezogen auf die Menge des ursprünglich [in der Verfahrensstufe (a)] verbrauchten Acrylnitrils normalerweise mehr als 85%, bei optimalen Bedingungen mehr als 90%, beträgt.

Ein weiterer Aspekt der vorliegenden Erfindung besteht darin, dass man nach Erreichen des etwa 40 bis 80%igen Umsatzes in der Verfahrensstufe (a) die schwache Base durch Zugabe einer schwachen Säure neutralisiert, um auch in diesem Fall ein zweiphasiges organisch-wässriges Gemisch zu erhalten, welches anstelle der anfänglichen schwachen Base unter anderem die durch die Neutralisierung gebildete Base enthält; diese Base stellt bereits den in der späteren Verfahrensstufe (d) verwendeten basischen Katalysator dar, so dass eine spezielle Zugabe eines basischen Katalysators für die Verfahrensstufe (d) vermieden wird.

Das ganze Verfahren gemäss diesem Aspekt der vorliegenden Erfindung ist dadurch gekennzeichnet, dass man
(a') in einem Temperaturbereich von 80°C bis 150°C und bei einem Druck von 1 bar (0,1 MPa) bis 5 bar (0.5 MPa) Acrylnitril mit Wasser in einem Molverhältnis von 1 : 0,5 bis 1 : 20 in Gegenwart einer schwachen Base, welche eine anorganische oder organische Base mit einem pKₐ-Wert von 8 bis 12 ist, solange umsetzt, bis ein Umsatz im Bereich von 40% bis 80% erreicht worden ist, um ein zweiphasiges organisch-wässriges Gemisch zu erhalten, dessen organische Phase hauptsächlich aus Bis(cyanoethyl)ether, 3-Hydroxypropionitril sowie unreagiertem Acrylnitril besteht, und dessen wässrige Phase hauptsächlich aus einer wässrigen Lösung der schwachen Base besteht;
(b') nach Abkühlung des in (a') erhaltenen Gemisches die schwache Base durch Zugabe einer schwachen Säure neutralisiert;
(c') von dem in (b') erhaltenen Gemisch das Acrylnitril und das Wasser abdestilliert, um ein hauptsächlich aus Bis(cyanoethyl)ether, 3-Hydroxypropionitril und der durch die Neutralisierung gebildeten Base bestehendes Gemisch zu erhalten;
(d') das in (c') erhaltene Gemisch einer Pyrolyse in einem Temperaturbereich von 120°C bis 160°C und bei einem vermindertem Druck von 5 mbar (0,5 kPa) bis 500 mbar (50 kPa) in Gegenwart der bereits vorhandenen, durch die Neutralisierung gebildeten Base unterwirft, um ein hauptsächlich aus 3-Hydroxypropionitril und Acrylnitril bestehendes Gemisch zu erhalten; und
(e') von dem in (d') erhaltenen Gemisch das gewünschte 3-Hydroxypropionitril durch fraktionierte Destillation isoliert.

Auch in diesem Verfahren wird gewünschtenfalls, und sogar vorzugsweise, das in der Verfahrensstufe (c') und/oder in der Verfahrensstufe (e') abdestillierte Acrylnitril in die Stufe (a') eines kontinuierlich durchgeführten Gesamtverfahrens gemäss diesem Erfindungsaspekt zurückgeführt.

Die Bedingungen der Verfahrensstufen (a'), (c'), (d') und (e') entsprechen im allgemeinen denjenigen Bedingungen, welche oben im Zusammenhang mit den Verfahrensstufen (a), (c), (d) resp. (e) beschrieben sind. Dies gilt auch für die Verfahrensstufe (c') gegenüber der Verfahrensstufe (c), obwohl in der ersteren Stufe nicht nur Acrylnitril, sondern auch noch Wasser abdestilliert werden, sowie auch für die Verfahrensstufe (d') gegenüber der Verfahrensstufe (d), obwohl in der ersteren Stufe die Base, welche als basischer Katalysator dient, nach Durchführung der Verfahrensstufe (c') bereits im Gemisch von Bis(cyanoethyl)ether und Acrylnitril vorhanden ist. Ebenfalls gelten die verschiedenen Bedeutungen des Ausdrucks "hauptsächlich" in Bezug auf vorhandene Bestandteile der in den Verfahrensstufen (a'), (c') und (d') definierten Phasen bzw. Gemische, wie dieser Ausdruck im Zusammenhang mit dem erstem Erfindungsaspekt. (Verfahren mit Stufen (a) - (e)) jeweils erläutert ist.

Als schwache Säure, die man zur Neutralisierung der schwachen Base in der Verfahrensstufe (b') verwendet, wird insbesondere eine niedere (C₁₋₃) Carbonsäure, z.B. Ameisensäure, Essigsäure oder Propionsäure, verwendet. Beispielsweise wird im Falle der Verwendung von Natriumcarbonat oder -bicarbonat als schwacher Base und von Essigsäure als schwacher Säure die Base Natriumacetat durch die Neutralisierung gebildet. Vorzugsweise werden ein Gemisch von Natriumcarbonat und -bicarbonat als schwache Base und Essigsäure als schwache Säure im Verfahren gemäss diesem Aspekt der vorliegenden Erfindung verwendet.

Gewünschtenfalls kann das erhaltene 3-Hydroxypropionitril einer Hydrierung zu 3-Aminopropanol unterworfen werden. Zu diesem Zwecke können an sich bekannte Methoden verwendet werden, beispielsweise unter Verwendung von Raney Nickel als Katalysator in Methanol oder Ethanol und in Gegenwart von wasserfreiem Ammoniak (siehe beispielsweise die Schweizerische Patentschrift 244.837, J. Chem. Soc. 1946, 94 sowie JP-Kokai 9963/1989). Ammoniak verhindert grösstenteils die Bildung von sekundären Aminen. Die Hydrierung erfolgt zweckmässigerweise bei Temperaturen um etwa 100°C und bei Drücken von etwa 25 bis etwa 100 bar H₂ (etwa 2,5 bis etwa 10,0 MPa).

Die Erfindung wird durch die nachfolgenden Beispiele 1-3 veranschaulicht. Das weitere Beispiel 4, veranschaulicht nicht das erfindungsgemässe Verfahren, sondern die Hydrierung des erfindungsgemäss hergestellten 3-Hydroxypropionitrils zu 3-Aminopropanol.

### Beispiel 1

### Reaktion von Acrylnitril mit Wasser und Spaltung des gebildeten Bis(cyanoethyl)ethers in 3-Hydroxypropionitril und Acrylnitril (Durchprozesse)

### (a) Natriumcarbonat als Base

In einem Rührautoklaven wurden 159,2 g (3 Mol) Acrylnitril und eine Lösung von 1,6 g (15 mmol, 0,5 Mol%) Natriumcarbonat in 54 g (3 Mol) entionisiertem Wasser vorgelegt. Nun wurde der Autoklav mit Argon gespült und verschlossen und das Reaktionsgemisch bei 200 U/Min. innerhalb von 20 Minuten auf 94°C (Innentemperatur) aufgeheizt. Bei 94 - 95°C wurde dann während einer Stunde weitergerührt (Manteltemperatur 110°C fallend bis 98°C; Druck etwa 1 bar / 0,1 MPa). Dann wurde das auf etwa 5 - 10°C (Innentemperatur) abgekühlte gelbliche Zweiphasengemisch in einen Scheidetrichter transferiert und die wässrige Phase abgetrennt. Dies ergab 188 g einer organischen Phase folgenden Gehalts nach Gaschromatographie (GC) in Flächenprozent (Fl.%):

| | |
|---|---|
| 54% | Acrylnitril |
| 5% | 3-Hydroxypropionitril |
| 38% | Bis(cyanoethyl)ether |
| 1 % | Acrylamid |

sowie 26 g einer wässrigen Phase. Die wässrige Phase enthielt noch wenig von den obigen Produkten. Diese wurden für die Ausbeute-Berechnung vernachlässigt. Wunschgemäss kann allerdings diese wässrige Phase in der Verfahrensstufe (a) wiederverwendet werden, wodurch kein Produkt verloren geht. Von der organischen Phase (188 g) wurde bei 200 - 10 mbar / 20 - 1 kPa (Oelbad: 60°C bis maximal 100°C; Sdp. ≤ 35°C) das überschüssige Acrylnitril mit dem gelösten Wasser über eine verspiegelte 10 cm - Füllkörper-Kolonne abdestilliert und in eine auf -70°C gekühlte Vorlage eingeleitet. Dies ergab 80 g eines zweiphasigen Destillats, von welchem die obere Phase aus 66,1 g Acrylnitril und die untere Phase aus 13,9 g Wasser bestand. Korrigiert gemäss dem Umstand, dass sich bei 20°C 7,4% Acrylnitril in Wasser und 3,1% Wasser in Acrylnitril lösen, entspricht dies 64,8 g (1,22 Mol) Acrylnitril und 15,2 g Wasser, was einen Umsatz von 59,3% an Acrylnitril entspricht.

Für die Fragmentierung wurden zum obigen Rückstand im Sumpf 300 mg (etwa 0,3 Gew.%) Natriumacetat zugegeben. Die Badtemperatur wurde auf 130 - 135°C erhöht und der Druck reduziert, wobei die Fragmentierung einsetzte. Bei 8 - 9 mbar (0,8 - 0,9 kPa) wurden nun kontinuierlich über eine 10 cm - Vigreux-Kolonne die Produkte 3-Hydroxypropionitril, Acrylnitril und wenig Acrylamid ohne Fraktionierung in eine auf -70°C gekühlte Vorlage abdestilliert. Dies ergab 103 g eines farblosen Destillats folgenden Gehalts nach GC in Fl.%:

| | |
|---|---|
| 60% | Acrylnitril |
| 38% | 3-Hydroxypropionitril |
| 1% | Acrylamid |

Der Rückstand bestand aus 3,8 g Oel.

Für die fraktionierte Destillation von Acrylnitril und 3-Hydroxypropionitril wurde das obige Destillat (103 g) an einer verspiegelten 10 cm - Füllkörper-Kolonne unter vermindertem Druck fraktioniert destilliert. Es gab folgende zwei Fraktionen:
1. Fraktion: Sdp. ≤ 35°C / 180 bis 10 mbar (18 bis 1 kPa); Badtemperatur 50 - 120°C; Vorlage gekühlt auf -70°C + zusätzliche Kühlfalle (-70°C); ergab 52,1 g (0,98 Mol) Acrylnitril.
2. Fraktion: Sdp. 99 - 102°C / 10 mbar (1 kPa); Badtemperatur 120 - 150°C; ergab 50,2 g (86%, korr.) 3-Hydroxypropionitril als farbloses Oel; Gehalt nach GC in Fl.%:

| | |
|---|---|
| 96,6% | 3-Hydroxypropionitril |
| 2,6% | Acrylamid |

### (b) Natriumcarbonat + Natriumbicarbonat (1 : 1) als Base

In einem Rührautoklaven wurden 159,2 g (3 Mol) Acrylnitril und eine Lösung von 6,36 g (60 mmol, 2 Mol%) Natriumcarbonat und 5,04 g (60 mmol, 2 Mol%) Natriumbicarbonat in 90 g (5 Mol) entionisiertem Wasser vorgelegt. Nun wurde der Autoklav mit Argon gespült und verschlossen und das Reaktionsgemisch bei 200 U/Min. innerhalb von 25 Minuten auf 120°C Aussentemperatur aufgeheizt. Bei dieser Temperatur wurde nun 1,5 Stunden gerührt. Dies ergab eine Innentemperatur von maximal 115°C (Druck: 2,2 bar / 0,22 MPa). Dann wurde das Gemisch auf 20°C abgekühlt und in einen 500 ml - Scheidetrichter transferiert. Dies ergab 189,2 g einer farblosen, organischen (oberen) Phase folgenden Gehalts nach GC in Fl.%:

| | |
|---|---|
| 41% | Acrylnitril |
| 8,5% | 3-Hydroxypropionitril |
| 50% | Bis(cyanoethyl)ether |
| 0,5% | Acrylamid |

und 71,2 g einer leicht gelblichen wässrigen (unteren) Phase. Von der organischen Phase (189,2 g) wurde nun bei 160 - 7 mbar / 16 - 0,7 kPa (Oelbad: 60°C bis maximal 100°C; Sdp. ≤ 35°C) das überschüssige Acrylnitril mit dem gelösten Wasser über eine verspiegelte 10 cm - Füllkörper-Kolonne abdestilliert und in eine auf -70°C gekühlte Vorlage sowie eine zusätzliche Kühlfalle bei -70°C eingeleitet. Dies ergab 73,8 g eines zweiphasigen Destillats, von welchem die obere Phase aus 62,1 g Acrylnitril und die untere Phase aus 11,7 g Wasser bestand. Korrigiert gemäss dem Umstand, dass sich bei 20°C 7,4 % Acrylnitril in Wasser und 3,1% Wasser in Acrylnitril lösen, entspricht dies 61,0 g (1,15 Mol) Acrylnitril und 12,8 g Wasser, was einem Umsatz von 60,0% an Acrylnitril entspricht.

Für die Fragmentierung wurden zum obigen Rückstand im Sumpf (115 g: 65% 3-Hydroxypropionitril, 32% Bis(cyanoethyl)ether sowie etwa 1% Acrylamid) 350 mg (etwa 0,3 Gew.%) Natriumacetat zugegeben. Die Badtemperatur wurde auf 130 - 135°C erhöht und der Druck reduziert, wobei die Fragmentierung einsetzte. Bei 7 - 8 mbar (0,7 - 0,8 kPa) wurden nun kontinuierlich über eine 10 cm - Vigreux-Kolonne die Produkte 3-Hydroxypropionitril, Acrylnitril und wenig Acrylamid ohne Fraktionierung zusammen in eine auf -70°C gekühlte Vorlage sowie eine zusätzliche Kühlfalle bei -70°C abdestilliert. Dies ergab 109 g eines farblosen Destillats folgenden Gehalts nach GC in Fl.%:

| | |
|---|---|
| 53% | Acrylnitril |
| 46% | 3-Hydroxypropionitril |
| 0,7% | Acrylamid |

Der Rückstand bestand aus 4 g Oel.

Für die fraktionierte Destillation von Acrylnitril und 3-Hydroxypropionitril wurde das obige Destillat (109 g) an einer verspiegelten 10 cm - Füllkörper-Kolonne unter vermindertem Druck fraktioniert destilliert. Es gab folgende zwei Fraktionen:
1. Fraktion: Sdp. 35°C / 180 bis 10 mbar (18 bis 1 kPa); Badtemperatur 50 - 120°C; Vorlage gekühlt auf -70°C + zusätzliche Kühlfalle (-70°C); ergab 50,0 g (0,94 Mol) Acrylnitril.
2. Fraktion: Sdp. 99 - 102°C / 10 mbar (1 kPa); ergab 57,5 g (87%, korr.) 3-Hydroxypropionitril als farbloses Oel; Gehalt nach GC in Gewichtsprozent (Gew.%):

| | |
|---|---|
| 97,8% | 3-Hydroxypropionitril |
| 1,8% | Acrylamid |

Der Rückstand bestand aus 1,5 g Oel.

### (c) Natriumbicarbonat als Base

In einem Rührautoklaven wurden 159,2 g (3 Mol) Acrylnitril und eine Lösung von 5,04 g (60 mmol, 2 Mol%) Natriumbicarbonat in 108 g (6 Mol) entionisiertem Wasser vorgelegt. Nun wurde der Autoklav mit Argon gespült und verschlossen und das Reaktionsgemisch bei 200 U/Min. innerhalb von 25 Minuten auf 125°C Aussentemperatur aufgeheizt. Bei dieser Temperatur wurde nun 80 Minuten weitergerührt. Dies ergab eine Innentemperatur von maximal 120 - 121°C (Druck: 3,1 bar / 0,31 MPa). Dann wurde das auf 15°C abgekühlte farblose Zweiphasengemisch in einen Scheidetrichter transferiert und die wässrige Phase abgetrennt. Dies ergab 173 g einer organischen (oberen) Phase folgenden Gehalts nach GC in Fl.%:

| | |
|---|---|
| 49,5% | Acrylnitril |
| 18,5% | 3-Hydroxypropionitril |
| 31% | Bis(cyanoethyl)ether |
| 0,7% | Acrylamid |

sowie 98,6 g einer wässrigen (unteren) Phase.

Zur Gewinnung der in der wässrigen Phase enthaltenen organischen Produkte 3-Hydroxypropionitril, Bis(cyanoethyl)ether, Acrylnitril und Acrylamid wurde das Wasser bei 180 - 8 mbar / 18 - 0,8 kPa über eine verspiegelte 10 cm - Füllkörper-Kolonne abdestilliert. Dies ergab 78,3 g Wasser (welches noch gelöstes Acrylnitril (Dest. 1) enthielt und 19,6 g Rückstand (gelbliches Oel mit festem anorganischem Salz). Dieser wurde einmal mit 50 ml und dreimal mit 25 ml, total 125 ml, Diethylether extrahiert und die organische Phase eingeengt, was 14 g eines öligen Extraktes ergab.

Die oben erwähnte organische Phase (173 g) wurde nun mit dem obigen Extrakt (14 g) vereinigt. Von der vereinigten organischen Phase (187 g) wurde nun das überschüssige Acrylnitril mit dem gelösten Wasser bei 200 - 10 mbar / 20 - 0,1 kPa (Oelbad: 60 bis maximal 100°C; Sdp. ≤ 35°C) über eine verspiegelte 10 cm - Füllkörper-Kolonne abdestilliert und in einer auf -70°C gekühlten Vorlage sowie einer zusätzlichen Kühlfalle bei -70°C aufgefangen. Dies ergab 107 g eines Rückstands und 79,4 g eines zweiphasigen Destillats, das aus Acrylnitril und Wasser (Dest. 2) bestand. Die beiden Destillate Dest. 1 und Dest. 2 (78,3 g + 79,4 g = 157,7 g) wurden vereinigt, im Scheidetrichter bei Raumtemperatur kurz geschüttelt und abgetrennt. Dies ergab eine obere Phase (64,5 g Acrylnitril) und eine untere Phase (93,2 g Wasser). Korrigiert gemäss dem Umstand, dass sich bei 20°C 7,4% Acrylnitril in Wasser und 3,1% Wasser in Acrylnitril lösen, entspricht dies 68,7 g (1,29 Mol) Acrylnitril und 89,0 g Wasser, was einem Umsatz von 57% an Acrylnitril entspricht.

Für die Fragmentierung wurden zum obigen Destillationsrückstand (107 g) 400 mg (etwa 0,4 Gew.%) Natriumacetat zugegeben. Die Badtemperatur wurde auf 130 - 135°C erhöht und der Druck reduziert, wobei die Fragmentierung einsetzte. Bei 8 - 9 mbar (0,8 - 0,9 kPa) wurden nun kontinuierlich über eine 10 cm - Vigreux-Kolonne die Produkte 3-Hydroxypropionitril, Acrylnitril und wenig Acrylamid ohne Fraktionierung in eine auf - 70°C gekühlte Vorlage sowie eine zusätzliche Kühlfalle bei -70°C abdestilliert. Dies ergab 103 g eines farblosen Destillats folgenden Gehalts nach GC in Fl.%:

| | |
|---|---|
| 46,5% | Acrylnitril |
| 52,5% | 3-Hydroxypropionitril + Acrylamid |

Der Rückstand bestand aus 4 g Oel.

Für die fraktionierte Destillation von Acrylnitril und 3-Hydroxypropionitril wurde das obige Destillat (103 g) an einer 10 cm - Füllkörper-Kolonne unter vermindertem Druck fraktioniert destilliert. Es gab folgende zwei Fraktionen:
1. Fraktion: Sdp. ≤ 35°C / 180 bis 10 mbar (18 bis 0,1 kPa); Vorlage gekühlt auf -70°C; ergab 41,9 g (0,79 Mol) Acrylnitril.
2. Fraktion: Sdp. 99 - 102°C / 10 mbar (1 kPa) ergab 60,1 g (90%, korr.) 3-Hydroxypropionitril als farbloses Oel; Gehalt nach GC in Fl.%:

| | |
|---|---|
| 97,5% | 3-Hydroxypropionitril |
| 2,3% | Acrylamid |

### Beispiel 2

### Reaktion von Acrylnitril mit Wasser und Spaltung des gebildeten Bis(cyanoethyl)ethers in 3-Hydroxypropionitril und Acrylnitril (Druchprozesse mit Rezyklisierung von Acrylnitril und Wasser; 6 Zyklen, wovon nur der 1. und der 5. Zyklus im folgenden exemplifiziert sind)

### (a) Natriumcarbonat + Natriumbicarbonat als Base

### 1. Zyklus

In einem 0,5l - Rührautoklaven wurden bei Raumtemperatur 159,2 g (3 Mol) Acrylnitril und eine Lösung von 6,36 g (60 mmol, 2 Mol%) Natriumcarbonat und 5,04 g (60 mmol, 2 Mol%) Natriumbicarbonat in 90 g (5 Mol) entionisiertem Wasser vorgelegt. Nun wurde der Autoklav mit Argon gespült und verschlossen, und das Reaktionsgemisch wurde bei 200 U/ Min. gerührt und innerhalb von 20 Minuten auf 115°C Innentemperatur (schwankend von 114,5 bis 115,1°C) aufgeheizt. Bei dieser Temperatur wurde dann 75 Minuten gerührt (Manteltemperatur: 129°C - 117°C; Druck anfänglich etwa 3 bar / 0,3 MPa, schliesslich 2,8 bar / 0,28 MPa). Anschliessend wurde auf +6°C abgekühlt und das Gemisch in einen Scheidetrichter gegeben, die Wasserphase (WP 1) abgetrennt, die organische Phase wieder in den Rührautoklaven gegeben und diese mit 18 g (1 Mol) Wasser (WW 1) 15 Minuten bei +5°C gerührt. Nach der Phasentrennung im Scheidetrichter erhielt man eine organische Phase (OP 1) und eine zweite Wasserphase (WP 2):

| | |
|---|---|
| OP 1 (184,65 g): Gehalt nach GC: | 49 g (0,94 Mol) Acrylnitril |
| | 15,9 g (0,22 Mol) 3-Hydroxypropionitril |
| | 94,7 g (0,7 Mol) Bis(cyanoethyl)ether |
| | 0,9 g (12 mmol) Acrylamid |
| | |
| WP 1 (71,5 g): Gehalt nach GC: | 1,0 g (19 mmol) Acrylnitril |
| | 4,3 g (60 mmol) 3-Hydroxypropionitril |
| | 3,2 g (25 mmol) Bis(cyanoethyl)ether |
| | 0,2 g (3 mmol) Acrylamid |
| | |
| WP 2 (22,3 g): Gehalt nach GC: | 0,9 g (16 mmol) Acrylnitril |
| | 2,1 g (29 mmol) 3-Hydroxypropionitril |
| | 1,7 g (14 mmol) Bis(cyanoethyl)ether |
| | 0,2 g (3 mmol) Acrylamid |

Von der organischen Phase (OP 1: 184,65 g) wurde nun bei 180 - 8 mbar / 18 - 0,8 kPa (Oelbad: 60 bis maximal 100°C; Sdp.≤ 35°C) das überschüssige Acrylnitril enthaltend gelöstes Wasser über eine verspiegelte 10 cm - Füllkörper-Kolonne in eine auf -70°C gekühlte Vorlage sowie eine zusätzliche Kühlfalle bei -70°C abdestilliert. Dies ergab 68,3 g eines zweiphasigen Destillats (ACN 1 + Wasser) folgender Zusammensetzung:
Obere Phase: 51,6 g Acrylnitril
Untere, wässrige Phase: 16,7 g Wasser
Korrigiert nach dem Umstand, dass sich bei 20°C 7,4% Acrylnitril in Wasser und 3,1% Wasser in Acrylnitril lösen, entspricht dies 51,2 g Acrylnitril (ACN 1) und 17,1 g Wasser (WP 3), was einem Umsatz von 67% an Acrylnitril im 1. Zyklus entspricht.

Für die Fragmentierung wurde zum obigen gelblichen Destillationsrückstand nun 0,4 g (etwa 0,4 Gew.%) Natriumacetat zugegeben. Die Badtemperatur wurde auf 130 - 135°C erhöht und der Druck reduziert, wobei die Fragmentierung einsetzte. Bei 7 - 8 mbar / 0,7 - 0,8 kPa wurden nun kontinuierlich über eine 10 cm - Vigreux-Kolonne die Produkte 3-Hydroxypropionitril, Acrylnitril und wenig Acrylamid ohne Fraktionierung zusammen in eine auf -70°C gekühlte Vorlage sowie eine zusätzliche Kühlfalle bei -70°C abdestilliert. Dies ergab 111 g eines farblosen Destillats (OP 2) folgenden Gehalts nach GC:

| | |
|---|---|
| 43,3 g (0,82 Mol) | Acrylnitril |
| 61,6 g (0,87 Mol) | 3-Hydroxypropionitril |
| 0,8 g (12 mmol) | Acrylamid |

sowie 3,9 g eines Rückstandes.

Für die fraktionierte Destillation von Acrylnitril und 3-Hydroxypropionitril wurde das obige Destillat (111 g) nun an einer verspiegelten 10 cm - Füllkörper-Kolonne unter vermindertem Druck fraktioniert destilliert:
1. Fraktion: Sdp. ≤ 35 °C / 180 bis 10 mbar (18 bis 1 kPa); Badtemperatur 50 - 120°C; Vorlage gekühlt auf -70°C + zusätzliche Kühlfalle (-70°C); diese Fraktion bestand aus 46,1 g (0,87 Mol) Acrylnitril (ACN 2).
2. Fraktion. Sdp. 99 - 102°C / 10 mbar (1 kPa); Diese Fraktion bestand aus 62 g (67,8%; 0,85 Mol) 3-Hydroxypropionitril als farblosem Oel; Gehalt nach GC:

| | |
|---|---|
| 98,4% (61 g) | 3-Hydroxypropionitril |
| 1,6% | Acrylamid |

Der Rückstand wog 0,7 g.

Die Ausbeute an 3-Hydroxypropionitril in diesem 1. Zyklus ist erwartungsgemäss tief, da die in den Wasserphasen WP 1 und WP 2 gelösten 3-Hydroxypropionitril und Bis(cyanoethyl)ether nicht berücksichtigt wurden. Diese beiden Phasen wurden jedoch in den folgenden Zyklen wieder eingesetzt. Daher stieg die Ausbeute in den folgenden Zyklen jeweils langsam an und erreichte im 5. und 6. Zyklus die Gegend von 90% (Plateau).

### 5. Zyklus

In einem 0,51-Rührautoklaven wurden bei Raumtemperatur 162 g (3,05 Mol) Acrylnitril bestehend aus 67 g Acrylnitril vom 4. Zyklus (ACN 1), 45 g Acrylnitril vom 4. Zyklus (ACN 2) sowie 50 g weiterem Acrylnitril (zur Ergänzung auf 162 g) und 111,1 g Wasserphasen (enthaltend regenerierten Katalysator) bestehend aus
73,2 g WP 1 (vom 4. Zyklus),
23,7 g WP 2 (vom 4. Zyklus) sowie
14,2 g WP 3 (vom 4. Zyklus)
vorgelegt. Nun wurde der Autoklav mit wenig Argon gespült und verschlossen, und das Reaktionsgemisch wurde bei 200 U/Min. gerührt und innerhalb von 20 Minuten auf 115°C Innentemperatur (schwankend von 114,5 bis 115,1°C) aufgeheizt. Bei dieser Temperatur wurde dann 75 Minuten gerührt (Manteltemperatur 129°C - 117°C; Druck anfänglich etwa 3 bar / 0,3 MPa, schliesslich 2,8 bar / 0,28 MPa). Anschliessend wurde auf +15°C abgekühlt und das Gemisch in einen Scheidetrichter gegeben, die Wasserphase (WP 1) abgetrennt, die organische Phase wieder in den Rührautoklaven gegeben und diese mit 18 g (1 Mol) Wasser (WW 1) 15 Minuten bei +5°C gerührt. Nach der Phasentrennung im Scheidetrichter erhielt man eine organische Phase (OP 1) und eine zweite Wasserphase (WP 2):

| | |
|---|---|
| OP 1 (192,8 g): Gehalt nach GC: | 56 g (1,1 Mol) Acrylnitril |
| | 14,6 g (0,21 Mol) 3-Hydroxypropionitril |
| | 87 g (0,70 Mol) Bis(cyanoethyl)ether |
| | 1,0 g (13 mmol) Acrylamid |
| | |
| WP 1 (71,1 g): Gehalt nach GC | 1,3 g (25 mmol) Acrylnitril |
| | 4,4 g (61 mmol) 3-Hydroxypropionitil |
| | 3,0 g (24 mmol) Bis(cyanoethyl)ether |
| | 0,5 g (6 mmol) Acrylamid |
| | |
| WP 2 (23,6 g): Gehalt nach GC: | 0,9 g (16 mmol) Acrylnitril |
| | 2,0 g (27 mmol) 3-Hydroxypropionitril |
| | 1,5 g (11 mmol) Bis(cyanoethyl)ether |
| | 0,3 g (3 mmol) Acrylamid |

Von der organischen Phase (OP 1: 192,8 g) wurde nun bei 180 - 8 mbar / 18 - 0,8 kPa (Oelbad: 60 bis maximal 100°C; Sdp.≤ 35°C) das überschüssige Acrylnitril enthaltend gelöstes Wasser über eine verspiegelte 10 cm - Füllkörper-Kolonne in eine auf -70°C gekühlte Vorlage sowie eine zusätzliche Kühlfalle bei -70°C abdestilliert. Dies ergab 77,0 g eines zweiphasigen Destillats (ACN 1 + Wasser) folgender Zusammensetzung:
Obere Phase: 63,1 g Acrylnitril
Untere, wässrige Phase: 13,9 g Wasser.
Korrigiert nach dem Umstand, dass sich bei 20°C 7,4% Acrylnitril in Wasser und 3,1% Wasser in Acrylnitril lösen, entspricht dies 62 g Acrylnitril (ACN 1) und 15 g Wasser (WP 3), was einen Umsatz von 62% an Acrylnitril im 5. Zyklus entspricht.

Für die Fragmentierung wurde zum obigen gelblichen Destillationsrückstand nun 0,4 g (etwa 0,4 Gew.%) Natriumacetat zugegeben. Die Badtemperatur wurde auf 130-135°C erhöht und der Druck reduziert, wobei die Fragmentierung einsetzte. Bei 7 - 8 mbar / 0,7 - 0,8 kPa wurden nun kontinuierlich über eine 10 cm - Vigreux-Kolonne die Produkte 3-Hydroxypropionitril, Acrylnitril und wenig Acrylamid ohne Fraktionierung zusammen in eine auf -70°C gekühlte Vorlage sowie eine zusätzliche Kühlfalle bei -70°C abdestilliert. Dies ergab 112,6 g eines farblosen Destillats (OP 2) folgenden Gehalts nach GC:

| | |
|---|---|
| 46,5 g (0,87 Mol) | Acrylnitril |
| 59,8 (0,84 Mol) | 3-Hydroxypropionitril |
| 0,8 g (11 mmol) | Acrylamid |

sowie 4,5 g eines Rückstandes

Für die fraktionierte Destillation von Acrylnitril und 3-Hydroxypropionitril wurde das obige Destillat (112,6 g) nun an einer verspiegelten 10 cm - Füllkörper-Kolonne unter vermindertem Druck fraktioniert destilliert:
1. Fraktion: Sdp. ≤ 35°C / 180 bis 10 mbar (18 bis 1 kPa); Badtemperatur 50 - 120 C; Vorlage gekühlt auf -70°C + zusätzliche Kühlfalle (-70°C); diese Fraktion bestand aus 50,5 g (0,95 Mol) Acrylnitril (ACN 2).
2. Fraktion: Sdp. 99 - 102°C / 10 mbar (1 kPa); diese Fraktion bestand aus 60,5 g (0,84 Mol, 90%, korr.) 3-Hydroxypropionitril als farblosem Oel; Gehalt nach GC:

| | |
|---|---|
| 98, 5% | 3-Hydroxypropionitril |
| 1,5% | Acrylamid |

Der Rückstand wog 0,9 g.

### b) Natriumbicarbonat als Base

### 1. Zyklus

In einem Rührautoklaven wurden bei Raumtemperatur 159,2 g (3 Mol) Acrylnitril und eine Lösung von 5,04 g (60 mmol, 2 Mol%) Natriumbicarbonat in 90 g (5 Mol) entionisiertem Wasser vorgelegt. Nun wurde der Autoklav mit Argon begast und verschlossen, und das Reaktionsgemisch wurde bei 200 U/Min. gerührt und innerhalb von 20 Minuten auf 120°C ± 0,5°C Innentemperatur aufgeheizt. Bei dieser Temperatur wurde dann 90 Minuten gerührt (Manteltemperatur 134°C - 122°C; Druck anfänglich etwa 3,3 bar / 0,33 MPa, schliesslich 2,9 bar / 0,29 MPa).

Anschliessend wurde auf +2°C abgekühlt und das Gemisch in einen Scheidetrichter gegeben, die Wasserphase (WP 1) abgetrennt, die organische Phase wieder in den Rührautoklaven gegeben und diese mit 18 g (1 Mol) Wasser (WW 1) 15 Minuten bei +5°C gerührt. Nach der Phasentrennung im Scheidetrichter erhielt man eine organische Phase (OP 1) und eine zweite Wasserphase (WP 2):

| | |
|---|---|
| OP 1 (166,65 g): Gehalt nach GC: | 64,9 g (1,2 Mol) Acrylnitril |
| | 11,4 g (0,16 Mol) 3-Hydroxypropionitril |
| | 74,1 g (0,60 Mol) Bis(cyanoethyl)ether |
| | 1,0 g (14 mmol) Acrylamid |
| | |
| WP 1 (77,6 g): Gehalt nach GC: | 3,1 g (58 mmol) Acrylnitril |
| | 7,2 g (0,1 mmol) 3-Hydroxypropionitril |
| | 3,1 g (24 mmol) Bis(cyanoethyl)ether |
| | 1,35 g (19 mmol) Acrylamid |
| | |
| WP 2 (23,8 g): Gehalt nach GC: | 1,1 g (21 mmol) Acrylnitril |
| | 2,0 g (28 mmol) 3-Hydroxypropionitril |
| | 1,0 g (18 mmol) Bis(cyanoethyl)ether |
| | 0,4 g (35 mmol) Acrylamid |

Von der organischen Phase (OP 1: 166,5 g) wurde nun bei 180 - 8 mbar / 18 - 0,8 kPa gleich wie bei den vorherigen Zyklen das überschüssige Acrylnitril abdestilliert. Dies ergab 69,7 g eines zweiphasigen Destillats (ACN 1 + Wasser) folgender Zusammensetzung:
Obere Phase: 62 g Acrylnitril
Untere Phase: 7,7 g Wasser.
Korrigiert nach dem Umstand, dass sich bei 20°C 7,4 % Acrylnitril in Wasser und 3,1% Wasser in Acrylnitril lösen entspricht dies 60,4 g Acrylnitril (ACN 1) und 9,3 g Wasser, was einen Umsatz von 62% an Acrylnitril entspricht.

Für die Fragmentierung wurde zum obigen gelblichen Destillationsrückstand nun 0,4 g (etwa 0,4 Gew.%) Kaliumacetat zugegeben und wie bei den vorherigen Zyklen fragmentiert. Dies ergab 86,5 g eines farblosen Destillats (OP 2) folgenden Gehalts nach GC:
38,3 g (0,72 Mol) Acrylnitril
47,6 g (0,67 Mol) 3-Hydroxypropionitril
0,5 g (7 mmol) Acrylamid
sowie 4,8 g eines Rückstandes.

Für die fraktionierte Destillation von Acrylnitril und 3-Hydroxypropionitril wurde das obige Destillat (86,5 g) nun wie bei den vorherigen Zyklen fraktioniert:
1. Fraktion: Sdp. *≤* 35°C / 180 bis 10 mbar (18 bis 0,1 kPa); diese Fraktion bestand aus 40,3 g (0,76 Mol) Acrylnitril (ACN 2).
2. Fraktion: 48,0 g (60%, 0,66 Mol, korr.) 3-Hydroxypropionitril als farbloses Oel; Gehalt nach GC:
   98,2% 3-Hydroxypropionitril sowie
   1,6% Acrylamid.
Der Rückstand wog 0,6 g.

### 5. Zyklus

In einem 0,5 l - Rührautoklaven wurden bei Raumtemperatur 160,1 g (3,02 Mol) Acrylnitril bestehend aus 75,1 g Acrylnitril (regeneriert) vom 4. Zyklus (ACN 1), 40,0 g Acrylnitril (regeneriert) vom 4. Zyklus (ACN 2), sowie 45,0 g weiterem Acrylnitril (zur Ergänzung) und 119,2 g Wasserphasen (WP, enthaltend regenerierten Katalysator) bestehend aus
83,7 g WP 1 (vom 4. Zyklus),
24,6 g WP 2 (vom 4. Zyklus) sowie
10,9 g WP 3 (vom 4. Zyklus)
vorgelegt. Nun wurde der Autoklav mit wenig Argon begast und verschlossen, und das Reaktionsgemisch wurde bei 200 U/Min. innerhalb von 20 Minuten auf 120°C ± 0,5°C Innentemperatur aufgeheizt und bei dieser Temperatur gerührt (Manteltemperatur 134°C - 122°C; Druck anfänglich etwa 3,3 bar / 0,33 MPa, schliesslich 2,9 bar / 0,29 MPa). Anschliessend wurde auf +2°C abgekühlt und das Gemisch in einen Scheidetrichter gegeben, die Wasserphase (WP 1) abgetrennt, die organische Phase wieder in den Rührautoklaven gegeben und diese mit 18 g (1 Mol) Wasser (WW 1) 15 Minuten bei +5°C gerührt. Nach der Phasentrennung im Scheidetrichter erhielt man eine organische Phase (OP 1) und eine zweite Wasserphase (WP 2):

| | |
|---|---|
| OP 1 (181,6 g): Gehalt nach GC: | 79,7 g (1,5 Mol) Acrylnitril |
| | 9,8 g (0,14 Mol) 3-Hydroxypropionitril |
| | 75,2 g (0,61 Mol) Bis(cyanoethyl)ether |
| | 1,8 g (25 mmol) Acrylamid |
| | |
| WP 1 (87,2 g): Gehalt nach GC: | 4,8 g (9 mmol) Acrylnitril |
| | 6,3 g (88 mmol) 3-Hydroxypropionitril |
| | 3,2 g (25 mmol) Bis(cyanoethyl)ether |
| | 2,6 g (36 mmol) Acrylamid |
| | |
| WP 2 (24,5 g): Gehalt nach GC: | 1,3 g (24 mmol) Acrylnitril |
| | 1,6 g (22 mmol) 3-Hydroxypropionitril |
| | 1,0 g (7 mmol) Bis(cyanoethyl)ether |
| | 0,7 g (9 mmol) Acrylamid |

Von der organischen Phase (OP 1: 181,6 g) wurde nun bei 180 - 8 mbar / 18 - 0,8 kPa gleich wie bei den vorherigen Zyklen das überschüssige Acrylnitril abdestilliert. Dies ergab 90,0 g eines zweiphasigen Destillats (ACN1 + Wasser) folgender Zusammensetzung:
Obere Phase: 80,8 g Acrylnitril
Untere Phase: 9,2 g Wasser
Korrigiert nach dem Umstand, dass sich bei 20°C 7,4% Acrylnitril in Wasser und 3,1% Wasser in Acrylnitril lösen, entspricht dies 79,0 g Acrylnitril (ACN 1) und 11,0 g Wasser (WP 3), was einen Umsatz von 51% an Acrylnitril entspricht.

Für die Fragmentierung wurde zum obigen gelblichen Destillationsrückstand 0,4 g (etwa 0,4 Gew.%) Kaliumacetat zugegeben und wie bei den vorherigen Zyklen fragmentiert. Dies ergab 88,0 g eines farblosen Destillats (OP 2) folgenden Gehalts nach GC:

| | |
|---|---|
| 36,4 g (0,69 Mol) | Acrylnitril |
| 47,5 g (0,67 Mol) | 3-Hydroxypropionitril |
| 2,0 g (24 mmol) | Acrylamid |

sowie 4,4 g eines Rückstandes.

Für die fraktionierte Destillation von Acrylnitril und 3-Hydroxypropionitril wurde das obige Destillat (88,0 g) wie bei den vorherigen Zyklen fraktioniert:
1. Fraktion: Sdp ≤ 35°C / 180 bis 10 mbar (18 bis 1 kPa); diese Fraktion bestand aus 38,9 g (0,73 Mol) Acrylnitril (ACN 2).
2. Fraktion: Sdp. 99 - 102C / 10 mbar (1 kPa); diese Fraktion bestand aus 48,0 g (82%, 0,654 Mol; korr.) 3-Hydroxypropionitril als farbloses Oel; Gehalt nach GC:
   96,9% 3-Hydroxypropionitril sowie
   2,8% Acrylamid.
Der ölige Rückstand wog 0,5 g.

### Beispiel 3

### Spaltung von Bis(cyanoethyl)ether (Versuch ohne anfängliche Anwesenheit von 3-Hydroxypropionitril und Acrylnitril)

### a) Spaltung mit Calciumoxid als Katalysator

In einem mit Magnetrührer, aufgesetzer 15 cm - Vigreux-Kolonne, Claisen-Aufsatz mit absteigendem Kühler und auf -70°C gekühlter Vorlage (250 ml -Rundkolben) ausgestatteten 500 ml - Rundkolben wurden 124,2 g (1 Mol) Bis(cyanoethyl)ether und 1,24 g (1 Gew. %) Calciumoxid (als Spaltungskatalysator) vorgelegt. In einem bei 120°C erhitzten Oelbad wurde nun dieses Gemisch unter vermindertem Druck (13 - 10 mbar / 1,3 - 1,0 kPa) und unter Rühren erhitzt. Bei 110°C Innentemperatur begann die Pyrolyse, wobei die Produkte 3-Hydroxypropionitril und Acrylnitril kontinuierlich in die auf -70°C gekühlte Vorlage destillierten. Gegen Schluss der Reaktion wurde die Oelbadtemperatur auf 140°C erhöht. Die gesamte Reaktionszeit betrug etwa 2 Stunden. Dabei wurden 121,3 g farbloses Destillat erhalten, welches nach GC aus 54% Acrylnitril, 45% 3-Hydroxypropionitril sowie 0,4% Bis(cyanoethyl)ether bestand. Der Rückstand bestand aus 4,1 g einer öligen Suspension ( Calciumoxid in polymerem Material).

Das obige Destillat (121,3 g) wurde einer fraktionierten Destillation unterworfen, und zwar unter Verwendung einer kleinen verspiegelten Füllkörper-Kolonne, etwa 15 cm lang. Bei einer Oelbadtemperatur von 60°C und einem Druck von 200 mbar / 20 kPa wurden bei einem Sdp. von ≤ 35°C als 1. Fraktion 52,9 g (100%ige Ausbeute) Acrylnitril (Gehalt nach GC 100%) als farblose Flüssigkeit erhalten. Als 2. Fraktion wurden bei einem Sdp. von 103 - 106°C / 13 mbar (1,3 kPa) 65,6 g (92 %, korr.) 3-Hydroxypropionitril als farblose Flüssigkeit erhalten; nach GC 99,5%; ¹H-NMR (CDCI₃): 2,62 (t, J=8 Hz, 2 H), 3,0 (bs, OH), 3,88 (t, J= 8 Hz, 2H); IR (Film, cm⁻¹): 3434 (OH), 2253 (CN).

| Mikroanalyse | | |
|---|---|---|
| Ber.: C 50,69% | H 7,09% | N 19,71% |
| Gef.: C 50,85% | H 7,10% | N 19,98% |

Der Rückstand bestand aus 2,1 g eines hellgelben wasserlöslichen Oels.

### (b) Spaltung mit Natriumacetat als Katalysator

In einem mit Magnetrührer, aufgesetzer 15 cm - Vigreux-Kolonne, Claisen-Aufsatz mit absteigendem Kühler und auf -70°C und gekühlter Vorlage (250 ml - Rundkolben) ausgestatteten 500 ml -Rundkolben wurden 124,2 g (1 Mol) Bis(cyanoethyl)ether und 1,24 g (1 Gew. %) wasserfreies Natriumacetat (als Spaltungskatalysator) vorgelegt. Bei einer Oelbadtemperatur von 120 - 140°C wurde nun bei 13 - 10 mbar / 1,3 - 1,0 kPa wie unter (a) beschrieben fragmentiert. Dies ergab 121,1 g eines farblosen Destillates, welches nach GC aus 57% Acrylnitril und 43% 3-Hydroxypropionitril bestand. Der Rückstand bestand aus 3,0 g einer hellgelben, öligen Suspension (Natriumacetat in polymerem Material).

Das obige Destillat (121,1 g) wurde nun einer fraktionierten Destillation unterworfen, und zwar unter Verwendung einer kleinen verspiegelten Füllkörper-Kolonne, etwa 15 cm lang. Bei einer Oelbadtemperatur von 60°C und einem Druck von 200 mbar /20 kPa, schliesslich 20 mbar (2 kPa) wurden bei einem Sdp. von ≤ 35°C als 1. Fraktion 55,8 g (>100%) Acrylnitril als farblose Flüssigkeit erhalten. Als 2. Fraktion wurden bei einem Sdp. von 103 - 106°C / 13 mbar (1,3 kPa) 64,5 g (90,0% korr.) 3-Hydroxypropionitril als farblose Flüssigkeit erhalten; Gehalt nach GC: 99,0%. Der Rückstand bestand aus 0,4 g eines farblosen Oels.

### Beispiel 4

### Hydrierung von 3-Hydroxypropionitril zu 3-Aminopropanol

### (a) Methanol/Ammoniak, 75 bar (7,5 MPa) Wasserstoff

In einem mit Begasungsrührer ausgestatteten 500 ml - Stahlrührautoklaven wurden 100 g (1,39 Mol, 99% nach GC) 3-Hydroxypropionitril in 100 ml Methanol vorgelegt. Die Lösung wurde nun mit 60 g gasförmigem Ammoniak gesättigt. Anschliessend wurden 10 g Raney-Nickel zugegeben und nach Spülung mit Stickstoff der Autoklav verschlossen. Nun wurden 22 bar (2,2 MPa) Wasserstoff aufgedrückt und innert einer Stunde das Gemisch auf 100°C erwärmt. Unter Erhöhung des Wasserstoffdrucks auf 75 bar (7,5 MPa) wurde bei 100°C weiterhydriert. Nach Abkühlung wurde der Autoklavinhalt über 25 g Speedex-Dicalite filtriert und mit Methanol nachgespült. Der grösste Teil des Methanols wurde bei bis etwa 25 mbar (2,5 kPa) / 40°C destillativ entfernt. Dies ergab 108 g eines blaufarbigen Rückstands, der noch etwas Methanol enthielt. Destillation über eine 10 cm - Füllkörper-Kolonne bei 2 - 3 mbar (0,2 - 0,3 kPa) ergab 95 g (86,5%, korr.) 3-Aminopropanol als farbloses Destillat, Sdp. 59 - 63°C / 2 - 3 mbar (0,2 - 0,3 kPa); Gehalt nach GC: 95,3%; ¹H-NMR (CDCl₃, 400 MHz): 1,68 (quint., J=6 Hz, 2H), 2,97, 2,3 (b, 3H), 2,97 (t, J=7Hz, 2H), 3,81 (t, J=6 Hz,, 2H); IR (Film, cm ¹): 3350 (b, OH, NH₂), 1050 (J).

| Mikroanalyse | | |
|---|---|---|
| Ber.: C 47,97% | H 12,08 | N18,65% |
| Gef.: C 47,77% | H 11,89% | N 18,52% |
| (H₂O: 0,61%) | | |

Der Rückstand wog 6,5 g.

### (b) Methanol/Ammoniak, 50 bar (50 MPa) Wasserstoff

100 g (1,39 Mol; 99% nach GC) 3-Hydroxypropionitril wurden in 100 ml Methanol vorgelegt. Die Lösung wurde bei 15 - 20°C mit 25 g gasförmigem Ammoniak gesättigt und nachher in einen mit Begasungsrührer ausgestatteten 500 ml - Stahlrührautoklaven transferiert. Dann wurden 10 g Raney-Nickel zugegeben und nach Spülung mit Stickstoff der Autoklav verschlossen. Es wurden 22 bar (2,2 MPa) Wasserstoff aufgedrückt und innert etwa einer Stunde auf 100°C erwärmt. Nun wurde der Wasserstoffdruck auf 50 bar (5,0 MPa) erhöht und das 3-Hydroxypropionitril bei 100°C noch 5 Stunden weiterhydriert. Nach Abkühlung wurde das Gemisch über 25 g Speedex-Dicalite filtriert, mit Methanol nachgespült und der grösste Teil vom Methanol unter vermindertem Druck entfernt. Dies ergab 104 g eines blaugrünen Rückstands. Destillation über eine 10 cm - Füllkörper-Kolonne bei 2 - 3 mbar / 0,2 - 0,3 kPa ergab 89,7 g (81%, korr.) 3-Aminopropanol als farbloses Oel, Sdp. 60,2 - 64°C / 2 - 3 mbar (0,2 - 0,3 kPa). Das Oel kristallisierte bei etwa 0°C. Gehalt nach GC: 93,6%; ¹H-NMR (CDCI₃, 400 MHz): 1,68 (quint., J= 6 Hz, 2H), 2,27 (b, OH, NH₂, 3H), 2,97 (t, J = 7Hz, 2H), 3,81 (t, J=6 Hz, 2H).

| Mikroanalyse | | |
|---|---|---|
| Ber.: C 47,97% | H 12,08% | N 18,65% |
| Gef.: C 48,43% | H 11,94% | N 18,76% |
| (H₂O: 1,53%) | | |

### (c) Ammoniak, 50 bar (5,0 MPa) Wasserstoff

In einem mit Begasungsrührer ausgestatteten 500 ml - Stahlrührautoklaven wurden 200 g (2,78 Mol, 99% nach GC) 3-Hydroxypropionitril vorgelegt, 20 g Raney-Nickel zugegeben und nach Verschliessen des Autoklaven 60 g Ammoniak aus einer Stahlflasche hineingepresst. Nach dem zusätzlichen Aufdrücken von 22 bar (2,2 MPa) Wasserstoff wurde auf 100°C erwärmt, der Wasserstoffdruck auf 50 bar (5,0 MPa) erhöht und 4 Stunden bei dieser Temperatur hydriert. Nach Abkühlung wurde das Ammoniak abgedampft und der Rückstand mit Methanol über 25 g Speedex-Dicalite filtriert, mit Methanol nachgespült und der grösste Teil vom Methanol unter vermindertem Druck entfernt. Dies ergab 211 g eines grünblauen, öligen Rückstands, den man über eine 10 cm - Füllkörper-Kolonne bei 2 - 3 mbar / 0,2 - 0,3 kPa destillierte. Auf diese Weise erhielt man 182,2 g (83%, korr.) 3-Aminopropanol als farbloses Oel, Sdp. 60 - 62,3°C / 2 - 3 mbar (0,2 - 0,3 kPa). Das Oel kristallisierte bei etwa 0°C. Gehalt nach GC: 95,1%; ¹H-NMR (CDCl₃, 400 MHz): identisch mit dem vorherigen unter (b) angegebenen Spektrum.

| Mikroanalyse | | |
|---|---|---|
| Ber.: C 47,97% | H 12,08% | N 18,65% |
| Gef.: C 48,14% | H 11,97% | N 18,87% |
| (H₂O: 1,17%) | | |

### (d) Isopropanol, 50 bar (5,0 MPa) Wasserstoff

In einem mit Begasungsrührer ausgestatteten 500 ml - Stahlrührautoklaven wurden 100 g (1,39 Mol, 99% nach GC) 3-Hydroxypropionitril in 100 g Isopropanol vorgelegt. Unter Begasung mit Stickstoff gab man 10 g Raney-Nickel zu und verschliess den Autoklaven. Nun wurde 7 Stunden bei 100°C und 50 bar / 5,0 MPa Wasserstoffdruck hydriert. Eine Aufarbeitung wie in den vorigen Varianten (a) - (c) ergab 112 g eines grunblauen, öligen Rückstands, den man wie üblich bei 2 - 3 mbar / 0,2 - 0,3 kPa destillierte. Auf diese Weise erhielt man 71,7 g (57,6%, korr) 3-Aminopropanol als farbloses Oel, Sdp. 61,3 - 64,7°C / 2 - 3 mbar (0,2 - 0,3 kPa). Gehalt nach GC: 84,1%; ¹H-NMR (CDCl₃, 400 MHz): identisch mit dem unter (b) angegebenen Spektrum.

## Patentansprüche

1. Verfahren zur Herstellung von 3-Hydroxypropionitril, **dadurch gekennzeichnet, dass** man
(a) in einem Temperaturbereich von 80°C bis 150°C und bei einem Druck von 1 bar (0,1 MPa) bis 5 bar (0,5 MPa) Acrylnitril mit Wasser in einem MolVerhältnis von 1 : 0,5 bis 1 : 20 in Gegenwart einer schwachen Base, welche eine anorganische oder organische Base mit einem pKₐ-Wert von 8 bis 12 ist, solange umsetzt, bis ein Umsatz im Bereich von 40% bis 80% erreicht worden ist, um ein zweiphasiges organisch-wässriges Gemisch zu erhalten, dessen organische Phase hauptsächlich aus Bis(cyanoethyl)ether, 3-Hydroxypropionitril sowie unreagiertem Acrylnitril besteht, und dessen wässrige Phase hauptsächlich aus einer wässrigen Lösung der schwachen Base besteht;
(b) nach Abkühlung des in (a) erhaltenen Gemisches dessen wässrige Phase abtrennt;
(c) von der nach (b) verbleibenden organischen Phase das Acrylnitril abdestilliert, um ein hauptsächlich aus Bis(cyanoethyl)ether und 3-Hydroxypropionitril bestehendes Gemisch zu erhalten;
(d) das in (c) erhaltene Gemisch einer Pyrolyse in einem Temperaturbereich von 120°C bis 160°C und bei einem vermindertem Druck von 5 mbar (0,5 kPa) bis 500 mbar (50 kPa) in Gegenwart eines basischen Katalysators unterwirft, welcher von der Gruppe Calcium-, Magnesium-, Strontium-, Titan-, Eisen- und Zinkoxide; Alkalimetallacetate; Alkalimetallformiate; Alkalimetall- und Bariumcarbonate; Alkalimetallbicarbonate; Calcium- und Kupferhydroxide; Di- und Trinatriumphosphate; Natriumfluorid; Natriumsilikat; und hochsiedende Trialkylamine ausgewählt ist, um ein hauptsächlich aus 3-Hydroxypropionitril und Acrylnitril bestehendes Gemisch zu erhalten; und
(e) von dem in (d) erhaltenen Gemisch das gewünschte 3-Hydroxypropionitril durch fraktionierte Destillation isoliert.

2. Verfahren nach Anspruch 1, **dadurch gekennzeichnet, dass** man die in der Verfahrensstufe (b) abgetrennte wässrige Phase, welche den Hauptteil der in der Verfahrensstufe (a) verwendeten Base enthält, in die Verfahrensstufe (a) eines kontinuierlich durchgeführten Gesamtverfahrens zurückführt, um dort mit weiterem Acrylnitril reagieren zu lassen.

3. Verfahren nach Anspruch 1 oder 2, **dadurch gekennzeichnet, dass** man das in der Verfahrensstufe (c) und/oder (e) abdestillierte bzw. zurückbleibende Acrylnitril in die Verfahrensstufe (a) eines kontinuierlich durchgeführten Gesamtverfahrens zurückführt, um dort mit weiterem Wasser in Gegenwart einer schwachen Base reagieren zu lassen.

4. Verfahren nach einem der Ansprüche 1 bis 3, **dadurch gekennzeichnet, dass** die in der Verfahrensstufe (a) verwendete schwache Base ein Alkalimetallcarbonat, ein Alkalimetallbicarbonat, ein Gemisch von zwei oder mehreren dieser anorganischen Basen, oder eine schwache organische Base, vorzugsweise Natriumcarbonat, Kaliumcarbonat, ein Gemisch von Natriumcarbonat und -bicarbonat oder ein Gemisch von Kaliumcarbonat und -bicarbonat ist.

5. Verfahren nach einem der Ansprüche 1 bis 4, **dadurch gekennzeichnet, dass** die auf die Menge eingesetzten Acrylnitrils bezogene Menge der schwachen Base 0,1 bis 5 Mol%, vorzugsweise 0,5 bis 2 Mol%, beträgt.

6. Verfahren nach einem der Ansprüche 1 bis 5, **dadurch gekennzeichnet, dass** das Molverhältnis Acrylnitril : Wasser in der Verfahrensstufe (a) 1 : 3 bis 1 : 8, vorzugsweise 1 : 2 bis 1 : 4, beträgt.

7. Verfahren nach einem der Ansprüche 1 bis 6, **dadurch gekennzeichnet, dass** die Umsetzung des Acrylnitrils mit dem Wasser in der Verfahrensstufe (a) bei Temperaturen im Bereich von 100°C bis 130°C und bei einem Druck von 1 bar (0,1 MPa) bis 3 bar (0,3 MPa) erfolgt.

8. Verfahren nach einem der Ansprüche 1 bis 7, **dadurch gekennzeichnet, dass** als basischer Katalysator in der Verfahrensstufe (d) Calciumoxid, Magnesiumoxid, Natriumacetat, Kaliumacetat, Natriumformiat, Kaliumformiat, Natriumcarbonat, Kaliumcarbonat, Bariumcarbonat, Natriumbicarbonat, Kaliumbicarbonat, Calciumhydroxid, Trinatriumphosphat oder Natriumfluorid, insbesondere Calciumoxid, Natriumacetat oder Kaliumacetat, verwendet wird.

9. Verfahren nach einem der Ansprüche 1 bis 8, **dadurch gekennzeichnet, dass** die auf die Gesamtmenge des in der Verfahrensstufe (c) erhaltenen und in der Verfahrensstufe (d) eingesetzten Gemisches von Bis(cyanoethyl)ether und 3-Hydroxypropionitril bezogene Menge des basischen Katalysators 0,05 bis 10 Gewichtsprozent, vorzugsweise 0,1 bis 3 Gewichtsprozent, beträgt.

10. Verfahren nach einem der Ansprüche 1 bis 9, **dadurch gekennzeichnet, dass** die Pyrolyse der Verfahrensstufe (d) bei Temperaturen im Bereich von 130°C bis 150°C und bei einem Druck von 10 mbar (1 kPa) bis 400 mbar (40 kPa) erfolgt.

11. Verfahren zur Herstellung von 3-Hydroxypropionitril, **dadurch gekennzeichnet, dass** man
(a') in einem Temperaturbereich von 80°C bis 150°C und bei einem Druck von 1 bar (0,1 MPa) bis 5 bar (0,5 MPa) Acrylnitril mit Wasser in einem Molverhältnis von 1 : 0,5 bis 1 : 20 in Gegenwart einer schwachen Base, welche eine anorganische oder organische Base mit einem pKₐ-Wert von 8 bis 12 ist, solange umsetzt, bis ein Umsatz im Bereich von 40% bis 80% erreicht worden ist, um ein zweiphasiges organisch-wässriges Gemisch zu erhalten, dessen organische Phase hauptsächlich aus Bis(cyanoethyl)ether, 3-Hydroxypropionitril sowie unreagiertem Acrylnitril besteht, und dessen wässrige Phase hauptsächlich aus einer wässrigen Lösung der schwachen Base besteht;
(b') nach Abkühlung des in (a') erhaltenen Gemisches die schwache Base durch Zugabe einer schwachen Säure neutralisiert;
(c') von dem in (b') erhaltenen Gemisch das Acrylnitril und das Wasser abdestilliert, um ein hauptsächlich aus Bis(cyanoethyl)ether, 3-Hydroxypropionitril und der durch die Neutralisierung gebildeter Base bestehendes Gemisch zu erhalten;
(d') das in (c') erhaltene Gemisch einer Pyrolyse in einem Temperaturbereich von 120°C bis 160°C und bei einem vermindertem Druck von 5 mbar (0,5 kPa) bis 500 mbar (50 kPa) in Gegenwart der bereits vorhandenen, durch die Neutralisierung gebildeten Base unterwirft, um ein hauptsächlich aus 3-Hydroxypropionitril und Acrylnitril bestehendes Gemisch zu erhalten; und
(e') von dem in (d') erhaltenen Gemisch das gewünschte 3-Hydroxypropionitril durch fraktionierte Destillation isoliert.

## Claims

1. A process for the production of 3-hydroxypropionitrile, which process comprises
(a) reacting acrylonitrile with water in a molar ratio of 1 : 0.5 to 1 : 20 in the presence of a weak base, which is an inorganic or organic base with a pKₐ value of 8 to 12, in a temperature range of 80°C to 150°C and at a pressure of 1 bar (0.1 MPa) to 5 bar (0.5 MPa) until a conversion in the range of 40% to 80% has been achieved in order to obtain a two-phase organic-aqueous mixture, the organic phase of which consists mainly of bis(cyanoethyl) ether and 3-hydroxypropionitrile as well as a relatively small amount of unreacted acrylonitrile, and the aqueous phase of which consists mainly of an aqueous solution of the weak base;
(b) after cooling the mixture obtained in (a) separating its aqueous phase;
(c) distilling off the acrylonitrile from the organic phase remaining after (b) in order to obtain a mixture consisting mainly of bis(cyanoethyl) ether and 3-hydroxypropionitrile;
(d) subjecting the mixture obtained in (c) to a pyrolysis in a temperature range of 120°C to 160°C and at a reduced pressure of 5 mbar (0.5 kPa) to 500 mbar (50 kPa) in the presence of a basic catalyst selected from the group consisting of calcium, magnesium, strontium, titanium, iron and zinc oxides, alkali metal acetates, alkali metal formates, alkali metal and barium carbonates, alkali metal bicarbonates, calcium and copper hydroxides, di- and trisodium phosphates, sodium fluoride, sodium silicate and high boiling trialkylamines in order to obtain a mixture consisting mainly of 3-hydroxypropionitrile and acrylonitrile; and
(e) isolating the desired 3-hydroxypropionitrile by fractional distillation from the mixture obtained in (d).

2. A process according to claim 1, wherein the aqueous phase separated off in process step (b), which contains the majority of the base used in process step (a), is conducted back into process step (a) of a continuously operated overall process in order to react there with additional acrylonitrile.

3. A process according to claim 1 or 2, wherein the acrylonitrile distilled off or remaining behind in process step (c) and/or (e) is conducted back into process step (a) of a continuously operated overall process in order to react there with additional water in the presence of a weak base.

4. A process according to any one of claims to 3, **characterized in that** the weak base used in process step (a) is an alkali metal carbonate, an alkali metal bicarbonate, a mixture of two or more of these inorganic bases or a weak organic base, preferably sodium carbonate, potassium carbonate, a mixture of sodium carbonate and sodium bicarbonate or a mixture of potassium carbonate and potassium bicarbonate.

5. A process according to any one of claims 1 to 4, wherein the amount of weak base employed is 0.1 to 5 mol%, preferably 0.5 to 2 mol%, based on the amount of acrylonitrile.

6. A process according to any one of claims 1 to 5, wherein the acrylonitrile : water molar ratio in process step (a) is 1 : 3 to 1 : 8, preferably 1 : 2 to 1 : 4.

7. A process according to any one of claims 1 to 6, wherein the reaction of the acrylonitrile with the water in process step (a) is effected at temperatures in the range of 100°C to 130°C and at a pressure of 1 bar (0.1 MPa) to 3 bar (0.3 MPa).

8. A process according to any one of claims 1 to 7, wherein calcium oxide, magnesium oxide, sodium acetate, potassium acetate, sodium formate, potassium formate, sodium carbonate, potassium carbonate, barium carbonate, sodium bicarbonate, potassium bicarbonate, calcium hydroxide, trisodium phosphate or sodium fluoride, especially calcium oxide, sodium acetate or potassium acetate, is used as the basic catalyst in process step (d).

9. A process according to any one of claims 1 to 8, wherein the amount of the basic catalyst is 0.05 to 10 weight percent, preferably 0.1 to 3 weight percent, based on the total amount of the mixture of bis(cyanoethyl) ether and 3-hydroxypropionitrile obtained in process step (c) and employed in process step (d).

10. A process according to any one of claims 1 to 9, wherein the pyrolysis of process step (d) is effected at temperatures in the range of 130°C to 150°C and at a pressure of 10 mbar (1 kPa) to 400 mbar (40 kPa).

11. A process for the production of 3-hydroxypropionitrile, which process comprises
(a') reacting acrylonitrile with water in a molar ratio of 1 : 0.5 to 1 : 20 in the presence of a weak base, which is an inorganic or organic base with a pKₐ value of 8 to 12, in a temperature range of 80°C to 150°C and at a pressure of 1 bar (0.1 MPa) to 5 bar (0.5 MPa) until a conversion in the range of 40% to 80% has been achieved in order to obtain a two-phase organic-aqueous mixture, the organic phase of which consists mainly of bis(cyanoethyl) ether and 3-hydroxypropionitrile as well as a relatively small amount of unreacted acrylonitrile, and the aqueous phase of which consists mainly of an aqueous solution of the weak base;
(b') after cooling the mixture obtained in (a') neutralizing the weak base by the addition of a weak acid;
(c') distilling off the acrylonitrile and the water from the mixture obtained in (b') in order to obtain a mixture consisting mainly of bis(cyanoethyl) ether, 3-hydroxypropionitrile and the base formed by the neutralization;
(d') subjecting the mixture obtained in (c') to a pyrolysis in a temperature range of 120°C to 160°C and at a reduced pressure of 5 mbar (0.5 kPa) to 500 mbar (50 kPa) in the presence of the already present base formed by neutralization in order to obtain a mixture consisting mainly of 3-hydroxypropionitrile and acrylonitrile; and
(e') isolating the desired 3-hydroxypropionitrile by fractional distillation from the mixture obtained in (d').

## Revendications

1. Procédé pour la préparation de 3-hydroxypropionitrile, **caractérisé en ce que**
(a) on met à réagir dans une gamme de température de 80°C à 150°C et à une pression de 1 bar (0,1 MPa) à 5 bar (0,5 MPa) de l'acrylonitrile avec de l'eau dans un rapport molaire de 1:0,5 jusqu'à 1:20 en présence d'une base faible, qui est une base inorganique ou organique avec une valeur de pKa de 8 à 12, jusqu'à ce que l'on atteigne un rendement dans la gamme de 40% à 80%, pour obtenir un mélange organique-aqueux diphasé, dont la phase organique est constituée principalement de bis(cyanoéthyl)éther, de 3-hydroxypropionitrile et d'acrylonitrile n'ayant pas réagi, et dont la phase aqueuse est constituée principalement d'une solution aqueuse de la base faible ;
(b) après refroidissement du mélange obtenu en (a) on sépare la phase aqueuse ;
(c) on distille l'acrylonitrile de la phase organique restant après (b), pour obtenir un mélange constitué principalement de bis(cyanoéthyl)éther et de 3-hydroxypropionitrile ;
(d) on soumet le mélange obtenu en (c) à une pyrolyse dans une gamme de températures de 120°C à 160°C et à une pression réduite à 5 mbar (0,5 kPa) jusqu'à 500 mbar (50 kPa) en présence d'un catalyseur basique, qui est choisi dans le groupe des oxydes de calcium, magnésium, strontium, titane, fer et zinc ; des acétates de métaux alcalins ; des formiates de métaux alcalins ; des carbonates de métaux alcalins et de baryum ; des bicarbonates de métaux alcalins ; des hydroxydes de calcium et de cuivre ; des phosphates de di- et trisodium ; du fluorure de sodium ; du silicate de sodium ; et des trialkylamines à point de fusion élevé, pour obtenir un mélange constitué principalement de 3-hydroxypropionitrile et d'acrylonitrile ; et
(e) on isole du mélange obtenu en (d) le 3-hydroxypropionitrile souhaité par distillation fractionnée.

2. Procédé selon la revendication 1, **caractérisé en ce que** l'on réintroduit la phase aqueuse séparée à l'étape de procédé (b), qui contient la partie principale de la base utilisée dans l'étape de procédé (a), dans l'étape de procédé (a) d'un procédé global réalisé en continu, pour la mettre à réagir avec une autre quantité d'acrylonitrile.

3. Procédé selon la revendication 1 ou 2, **caractérisé en ce que** l'on réintroduit l'acrylonitrile distillé ou restant dans l'étape de procédé (c) et/ou (e) dans l'étape de procédé (a) d'un procédé global réalisé en continu, pour l'y mettre à réagir avec une autre quantité d'eau en présence d'une base faible.

4. Procédé selon l'une des revendications 1 à 3, **caractérisé en ce que** la base faible utilisée dans l'étape de procédé (a) est un carbonate de métal alcalin, un bicarbonate de métal alcalin, un mélange de deux ou plus de ces bases inorganiques, ou une base organique faible, de préférence du carbonate de sodium, du carbonate de potassium, un mélange de carbonate et de bicarbonate de sodium ou un mélange de carbonate et de bicarbonate de potassium.

5. Procédé selon l'une des revendications 1 à 4, **caractérisé en ce que** la quantité de la base faible rapportée à la quantité d'acrylonitrile mise en oeuvre s'élève à entre 0,1 et 5 % en moles, de préférence entre 0,5 et 2 % en moles.

6. Procédé selon l'une des revendications 1 à 5, **caractérisé en ce que** le rapport en moles acrylonitrile:eau dans l'étape de procédé (a) s'élève à 1:3 jusqu'à 1:8, de préférence de 1:2 à 1:4.

7. Procédé selon l'une des revendications 1 à 6, **caractérisé en ce que** l'on réalise la réaction de l'acrylonitrile avec l'eau dans l'étape de procédé (a) à des températures dans la gamme de 100°C à 130°C et à une pression de 1 bar (0,1 MPa) jusqu'à 3 bar (0,3 MPa).

8. Procédé selon l'une des revendications 1 à 7, **caractérisé en ce que** l'on utilise comme catalyseur basique dans l'étape de procédé (d) l'oxyde de calcium, l'oxyde de magnésium, l'acétate de sodium, l'acétate de potassium, le formiate de sodium, le formiate de potassium, le carbonate de sodium, le carbonate de potassium, le carbonate de baryum, le bicarbonate de sodium, le bicarbonate de potassium, l'hydroxyde de calcium, le phosphate de trisodium ou la fluorure de sodium, en particulier l'oxyde de calcium, l'acétate de sodium ou l'acétate de potassium.

9. Procédé selon l'une des revendications 1 à 8, **caractérisé en ce que** la quantité du catalyseur basique rapportée à la quantité globale du mélange de bis(cyanoéthyl)éther et de 3-hydroxypropionitrile obtenue à l'étape de procédé (c) et mise en oeuvre à l'étape de procédé (d) s'élève à entre 0,05 et 10 % en poids, de préférence entre 0,1 et 3 % en poids.

10. Procédé selon l'une des revendications 1 à 9, **caractérisé en ce que** l'on réalise la pyrolyse de l'étape de procédé (d) à des températures comprises dans la gamme de 130°C à 150°C et à une pression de 10 mbar (1 kPa) à 400 mbar (40 kPa).

11. Procédé pour la préparation de 3-hydroxypropionitrile, **caractérisé en ce que**
(a') on met à réagir dans une gamme de température de 80°C à 150°C et à une pression de 1 bar (0,1 MPa) à 5 bar (0,5 MPa) de l'acrylonitrile avec de l'eau dans un rapport molaire de 1:0,5 à 1:20 en présence d'une base faible, qui est une base inorganique ou organique avec une valeur de pKa de 8 à 12, jusqu'à ce que l'on obtienne un rendement dans la gamme de 40 % à 80 %, pour obtenir un mélangé organique-aqueux biphasé, dont la phase organique est constituée principalement de bis(cyanoéthyl)éther, de 3-hydroxypropionitrile et d'acrylonitrile n'ayant pas réagi, et dont la phase aqueuse est constituée principalement d'une solution aqueuse de la base faible;
(b') on neutralise après refroidissement du mélange obtenu en (a') la base faible par ajout d'un acide faible ;
(c') on distille du mélange obtenu en (b') l'acrylonitrile et l'eau, pour obtenir un mélange constitué principalement de bis(cyanoéthyl)éther, de 3-hydroxypropionitrile et de la base formée par la neutralisation ;
(d') on soumet le mélange obtenu en (c') à une pyrolyse dans une gamme de température de 120°C à 160°C et à une pression réduite à 5 mbar (0,5 kPa) jusqu'à 500 mbar (50 kPa) en présence de la base déjà présente, formée par la neutralisation, pour obtenir un mélange constitué principalement de 3-hydroxypropionitrile et d'acrylonitrile ; et
(e') on isole du mélange obtenu en (d') le 3-hydroxypropionitrile souhaité par distillation fractionnée.
